(19) Europäisches Patentamt
European Patent Office
Office européen des brevets

(11) Veröffentlichungsnummer : **0 440 582 A1**

(12) **EUROPÄISCHE PATENTANMELDUNG**

(21) Anmeldenummer : **91810055.3**

(22) Anmeldetag : **25.01.91**

(51) Int. Cl.[5] : **C07C 239/20**, C07C 259/04

(30) Priorität : **02.02.90 US 474422**

(43) Veröffentlichungstag der Anmeldung : **07.08.91 Patentblatt 91/32**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Schneider, Hans-Dieter, Dr.**
**Efringerstrasse 32**
**W-7858 Weil am Rhein (DE)**

(54) **Verfahren zur Herstellung von O-substituierten Hydroxylaminen.**

(57) O-substituierten Hydroxylamine der Formel I

$$H_2NOR_1 \quad (I)$$

worin
$R_1$ $C_1$-$C_6$-Alkyl, $C_1$-$C_6$-Halogenalkyl, $-CH_2CR_2{=}CR_3R_4$ oder $-CH_2C{\equiv}CR_5$ ;
$R_2$ Wasserstoff, Halogen oder Methyl ;
$R_2$ Wasserstoff, Halogen oder Methyl ;
$R_4$ Wasserstoff, Halogen oder Methyl ; und
$R_5$ Wasserstoff, Methyl oder Ethyl bedeuten,
werden hergestellt, indem man ein Nitril der Formel II

$$R_6\text{-}CN \quad (II)$$

worin $R_6$ für $C_1$-$C_4$-Alkyl, Phenyl oder Benzyl steht, mit einem Alkohol der Formel III

$$R_7\text{-}OH \quad (III)$$

worin $R_7$ für $C_1$-$C_5$-Alkyl, Benzyl oder $C_1$-$C_4$-Alkoxyethyl steht, in einem organischen mit Wasser nicht oder nur sehr begrenzt mischbaren Lösungsmittel in Gegenwart von Halogenwasserstoff HX umsetzt, die erhaltene Suspension des Iminoester-Hydrohalogenids der Formel IV

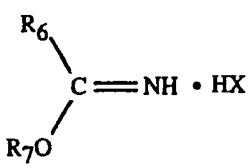

(IV)

worin $R_6$ und $R_7$ die oben angegebene Bedeutung haben und X für Chlor, Brom oder Jod steht, bei einer Temperatur von -3 bis +5°C in eine wässrige Suspension eines Carbonats oder Hydrogencarbonats eindosiert, der erhaltenen Mischung bei einer Temperatur von -20 bis -5°C eine wässrige Lösung eines Hydroxylaminsalzes zugibt, die wässrige Phase nach Erwärmung auf Raumtemperatur abtrennt, der erhaltenen Lösung des Hydroximsäureesters der Formel V

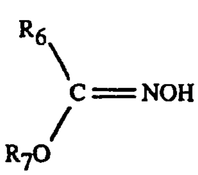

(V)

worin $R_6$ und $R_7$ die oben angegebene Bedeutung haben, in einem organischen Lösungsmittel zunächst eine wässrige Lösung einer starken Base und dann ein Alkylierungsmittel der Formel VI

$$Y\text{-}R_1 \quad (VI)$$

worin $R_1$ die oben angegebene Bedeutung hat und Y für Chlor, Brom, Jod oder $-O-SO_2R_8$, und $R_8$ für $C_1$-$C_4$-Alkyl, $C_1$-$C_4$-Halogenalkyl, Phenyl oder durch Phenyl substituiertes $C_1$-$C_4$-Alkyl steht, wobei der Phenylring seinerseits noch ein- bis dreifach durch Halogen oder $C_1$-$C_4$-Alkyl substituiert sein kann und Y auch für $-OSO_2OR_1$ steht, wenn $R_1$ $C_1$-$C_2$-Alkyl bedeutet, bei Raumtemperatur bis zur Siedetemperatur des Reaktionsgemisches zugibt, nach beendeter O-Alkylierung auf Raumtemperatur abkühlt, die wässrige Phase abtrennt, den gebildeten Oximether der Formel VII

EP 0 440 582 A1

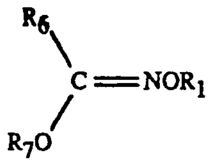

(VII)

worin $R_1$, $R_6$ und $R_7$ die oben angegebene Bedeutung haben, durch Verrühren mit einer wässrigen Lösung einer starken Säure hydrolysiert und die das Salz des O-substituierten Hydroxylamins der Formel I enthaltende wässrige Phase abtrennt, und dieses gegebenenfalls durch Verdampfen des Wassers isoliert.

# VERFAHREN ZUR HERSTELLUNG VON O-SUBSTITUIERTEN HYDROXYLAMINEN

Die vorliegende Erfindung betrifft ein Verfahren zur Herstellung von O-substituierten Hydroxylaminen der Formel I

$$H_2NOR_1 \quad (I)$$

worin
$R_1$ $C_1$-$C_6$-Alkyl, $C_1$-$C_6$-Halogenalkyl, $-CH_2CR_2{=}CR_3R_4$ oder $-CH_2C{\equiv}CR_5$ ;
$R_2$ Wasserstoff, Halogen oder Methyl ;
$R_3$ Wasserstoff, Halogen oder Methyl ;
$R_4$ Wasserstoff, Halogen oder Methyl ; und
$R_5$ Wasserstoff, Methyl oder Ethyl bedeuten,
sowie Zwischenprodukte, die bei diesem Verfahren durchlaufen werden.

Die O-substituierten Hydroxylamine der Formel I sind wertvolle Zwischenprodukte zur Herstellung von herbizid wirksamen Acylcyclohexandionen, wie sie beispielsweise in der europäischen Patentanmeldung Nr. 0 243 313 offenbart sind.

Herstellungsverfahren von O-substituierten Hydroxylaminen und deren Vorstufen sind in der Literatur beschrieben.

Aus USP 4,743,701 ist bekannt, Hydroximsäureester der Formel

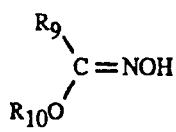

(VIII)

worin $R_9$ und $R_{10}$ unabhängig voneinander für Alkyl, Cycloalkyl, Aryl oder Aralkyl stehen, in der Weise herzustellen, indem man in erster Stufe ein Nitril der Formel $R_9$ CN unter wasserfreien Bedingungen mit einem Alkohol der Formel $R_{10}$-OH in einem organischen Lösungsmittel in Gegenwart von Halogenwasserstoff zum entsprechenden Imidat-Hydrohalogenid umsetzt, das feste Imidat-Hydrohalogenid von der Reaktionsmischung abtrennt und dieses anschliessend in zweiter Stufe unter wasserfreien Bedingungen in einem organischen Lösungsmittel mit einem Hydroxylaminsalz und mit Ammoniakgas zum Hydroximsäureester der Formel VIII umsetzt.

Aus der deutschen Offenlegungsschrift Nr. 2651083 ist bekannt, O-substituierte Hydroxylamine der Formel IX

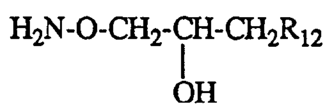

(IX)

worin $R_{12}$ für einen Ether-, Thiol-, Phenol- oder Thiophenolrest oder für eine Aminogruppe steht, herzustellen, indem man einen Hydroximsäureester der Formel X

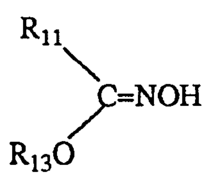

(X)

worin $R_{11}$ für einen Alkyl- oder Arylrest steht, mit 2-Propanolen der Formel XI

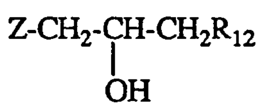

(XI)

worin Z für Halogen oder eine Sulfonestergruppierung steht, in nichtwässrigem Medium in Gegenwart einer

Base zu den O-substituierten Hydroximsäureestern der Formel XII

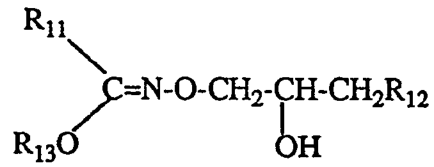

(XII)

umsetzt und anschliessend die Schutzgruppe hydrolytisch abspaltet.

Diese bekannten Verfahren weisen jedoch eine Vielzahl von Nachteilen auf. So muss beispielsweise für die Herstellung der Hydroximsäureester der Formel VIII gemäss USP 4,743,701 gasförmiges Ammoniak eingesetzt werden, wodurch als Nebenprodukt grosse Mengen unerwünschter Ammoniumsalze anfallen.
Neben der ökonomisch unvorteilhaften mehrstufigen Reaktionsführung fallen durch die wasserfreie Arbeitsweise im 2. Verfahrensschritt grosse Mengen an organischen Lösungsmitteln an, deren Rückführung oder Beseitigung zusätzlichen Aufwand erfordert

Die aus DOS 2651083 bekannten O-substituierten Hydroximsäureester der Formel XII erfordern ebenfalls zu ihrer Herstellung eine ökologisch ungünstige wasserfreie Arbeitsweise. Die für dieses Verfahren als Ausgangsstoffe benötigten Hydroximsäureester der Formel X müssen zuvor in einem getrennten Verfahren hergestellt werden.

Es wurde nun gefunden, dass man die O-substituierten Hydroxylamine der Formel I in guter Ausbeute und Reinheit auf ökonomisch und ökologisch besonders vorteilhafte Weise herstellen kann, wenn man
ein Nitril der Formel II

$R_6$-CN (II)

worin $R_6$ für $C_1$-$C_4$-Alkyl, Phenyl oder Benzyl steht, mit einem Alkohol der Formel III

$R_7$-OH (III)

worin $R_7$ für $C_1$-$C_5$-Alkyl, Benzyl oder $C_1$-$C_4$-Alkoxyethyl steht, in einem organischen mit Wasser nicht oder nur sehr begrenzt mischbaren Lösungsmittel in Gegenwart von Halogenwasserstoff HX umsetzt, die erhaltene Suspension des Iminoester-Hydrohalogenids der Formel IV

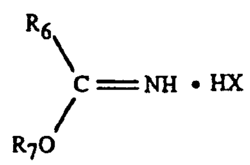

(IV)

worin $R_6$ und $R_7$ die oben angegebene Bedeutung haben und X für Chlor, Brom oder Jod steht, bei einer Temperatur von -3 bis +5°C in eine wässrige Suspension eines Carbonats oder Hydrogencarbonats eindosiert, der erhaltenen Mischung bei einer Temperatur von -20 bis -5°C eine wässrige Lösung eines Hydroxylaminsalzes zugibt, die wässrige Phase nach Erwärmung auf Raumtemperatur abtrennt, der erhaltenen Lösung des Hydroximsäureesters der Formel V

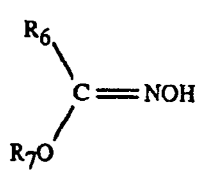

(V)

worin $R_6$ und $R_7$ die oben angegebene Bedeutung haben, in einem organischen Lösungsmittel zunächst eine wässrige Lösung einer starken Base und dann ein Alkylierungsmittel der Formel VI

Y-$R_1$ (VI)

worin $R_1$ die oben angegebene Bedeutung hat und Y für Chlor, Brom, Jod oder -O-$SO_2R_8$, und $R_8$ für $C_1$-$C_4$-Alkyl, $C_1$-$C_4$-Halogenalkyl, Phenyl oder durch Phenyl substituiertes $C_1$-$C_4$-Alkyl steht, wobei der Phenylring seinerseits noch ein- bis dreifach durch Halogen oder $C_1$-$C_4$-Alkyl substituiert sein kann und Y auch für -$OSO_2OR_1$ steht, wenn $R_1$ $C_1$-$C_2$-Alkyl bedeutet, bei Raumtemperatur bis zur Siedetemperatur des Reaktionsgemisches zugibt, nach beendeter O-Alkylierung auf Raumtemperatur abkühlt, die wässrige Phase abtrennt, den gebildeten Oximether der Formel VII

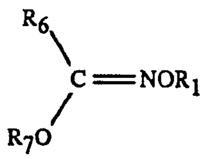

(VII)

worin $R_1$, $R_6$ und $R_7$ die oben angegebene Bedeutung haben, durch Verrühren mit einer wässrigen Lösung einer starken Säure hydrolysiert und die das Salz des O-substituierten Hydroxylamins der Formel I enthaltende wässrige Phase abtrennt, und dieses gegebenenfalls durch Verdampfen des Wassers isoliert.

Die in den Substituenten vorkommenden Alkylgruppen können geradkettig oder verzweigt sein und stehen beispielsweise für Methyl, Ethyl, n-Propyl, iso-Propyl, n-Butyl, iso-Butyl, sek.-Butyl, tert.-Butyl, n-Pentyl oder die isomeren Pentyl, n-Hexyl oder die isomeren Hexyl. Bevorzugt sind Alkylreste mit höchstens 4 Kohlenstoffatomen, insbesondere Methyl und Ethyl.

Die im Substituenten $R_6$ vorkommenden Phenyl- und Benzylgruppen können gegebenenfalls durch Halogen, Nitro, $C_1$-$C_4$-Alkyl oder $C_1$-$C_4$-Halogenalkyl substituiert sein wie beispielsweise 2-Chlorphenyl, 3-Chlorphenyl, 4-Chlorphenyl, 2,4-Dichlorphenyl, 4-Fluorphenyl, 4-Methylphenyl, 2,4-Dimethylphenyl, 4-Methylbenzyl, 4-Nitrobenzyl, 4-Trifluormethylphenyl oder 4-Brombenzyl.

Unter Alkoxy ist zu verstehen : Methoxy, Ethoxy, n-Propyloxy, iso-Propyloxy oder die vier isomeren Butyloxy, insbesondere aber Methoxy oder Ethoxy.

Unter Halogen selbst sowie als Teil eines Substituenten wie Halogenalkyl sind Fluor, Chlor, Brom und Jod, vorzugsweise jedoch Chlor und Brom zu verstehen. Halogenalkyl steht in der Regel für Chlormethyl, Fluormethyl, Difluormethyl, Trifluormethyl, 2-Chlorethyl, 2,2,2-Trifluorethyl, 1,1,2,2-Tetrafluorethyl, Pentafluorethyl, 1,1,2-Trifluor-2-chlorethyl, 2,2,2-Trifluor-1,1-dichlorethyl, Pentachlorethyl, 3,3,3-Trifluorpropyl, 2,3-Dichlorpropyl, 2-Chlorethyl und 3-Chlorpropyl, insbesondere aber Fluormethyl, Chlormethyl, Difluormethyl und Trifluormethyl.

Durch Phenyl substituierte $C_1$-$C_4$-Alkylgruppen sind beispielsweise 4-Phenylbutyl, 2-Phenylethyl oder Benzyl.

Durch Halogen oder $C_1$-$C_4$-Alkyl substituierte Phenylgruppen stehen beispielsweise für 4-Bromphenyl, 2,4-Dichlorphenyl, 2,4,6-Trichlorphenyl, 4-Fluorphenyl, 4-Methylphenyl, 2,4-Dimethylphenyl, 4-Ethylphenyl oder 4-iso-Propylphenyl. Bevorzugte durch obengenannte Reste substituierte Phenylgruppen sind 4-Bromphenyl und 4-Methylphenyl.

Als organisches, mit Wasser nicht oder nur sehr begrenzt (bis zu 10 Gew.%) mischbares Lösungsmittel haben sich für das erfindungsgemässe Verfahren Toluol, Hexan, Diethylether, Xylol, Cyclohexan, Nitrobenzol, Dichlorethan, Dichlormethan oder Trichlormethan als gut geeignet erwiesen. Ein ganz besonders bevorzugtes Lösungsmittel ist Toluol.

Unter den im erfindungsgemässen Verfahren zur Umsetzung mit dem Iminoester-Hydrohalogenid der Formel IV eingesetzten Carbonaten oder Hydrogencarbonaten sind insbesondere die Alkaliverbindungen zu verstehen wie Natriumcarbonat, Kaliumcarbonat, Natriumhydrogencarbonat oder Kaliumhydrogencarbonat. Besonders bevorzugt ist Natriumhydrogencarbonat.

Die Ausgangsstoffe der Formeln II und III sowie die Zwischenprodukte der Formeln IV, V und VI des erfindungsgemässen Verfahrens sind bekannt und teilweise im Handel erhältlich.

Die in erfindungsgemässen Verfahren durchlaufenen Zwischenprodukte der Formel VII sind mit Ausnahme derjenigen Verbindungen, in denen $R_1$ für $C_1$-$C_6$-Alkyl, $C_1$-$C_6$-Halogenalkyl oder Allyl steht, neu und bilden ebenfalls einen Gegenstand der vorliegenden Erfindung. Die Verbindungen der Formel VII können gewünschtenfalls zur Charakterisierung aus dem Reaktionsmedium isoliert werden. Falls gewünscht, können die Endprodukte der Formel I in freier Form durch Behandlung mit Base nach bekannten Methoden erhalten werden.

Die Umsetzung des Nitrils der Formel II mit dem Alkohol der Formel III zum Iminoester-Hydrohalogenid der Formel IV wird in der Regel bei Temperaturen von -10 bis +30°C, vorzugsweise bei 0 bis 25°C durchgeführt. Als Halogenwasserstoff HX wird dabei Chlorwasserstoff bevorzugt. Vor der weiteren Umsetzung kann das Imidat-Hydrohalogenid-Reaktionsgemisch gewünschtenfalls von nicht umgesetztem Chlorwasserstoff durch Evakuieren oder Einleiten von Stickstoff befreit werden.

Für das erfindungsgemässe Verfahren geeignete Hydroxylaminsalze sind beispielsweise das Hydroxylamin-Hydrochlorid, das Hydroxylaminsulfat sowie das Hydroxylaminphosphat, besonders bevorzugt ist das Hydroxylamin-Hydrochlorid sowie das Hydroxylaminsulfat.

Die Alkylierung des Hydroximsäureesters der Formel V wird bei Temperaturen von Raumtemperatur bis zur Siedetemperatur des Reaktionsgemisches durchgeführt. Ein bevorzugter Temperaturbereich liegt zwischen +50 und +80°C.
Für diesen Verfahrensschritt sind als starke Basen wässrige Carbonate und Hydroxide von Alkali- und Erdalkalimetallen geeignet.
Bevorzugte starke Basen sind wässriges Natriumhydroxid und wässriges Natriumcarbonat. Vorzugsweise werden der Hydroximsäureester der Formel V, das Alkylierungsmittel der Formel VI sowie die Base in äquimolaren Mengen eingesetzt.

Die Alkylierung wird mit Vorteil in Gegenwart katalytischer Mengen eines Phasentransferkatalysators durchgeführt. Der Phasentransferkatalysator wird vorteilhaft in einer Menge von 0,01 bis 10 Mol%, vorzugsweise 0,5 bis 1 Mol% bezogen auf die Verbindung der Formel V eingesetzt.

Als Phasentransferkatalysatoren sind generell quartäre Ammoniumsalze und Kronenäther geeignet. Bevorzugte Phasentransferkatalysatoren sind 18-Crown-6, Benzyltrimethylammoniumchlorid, Tetrabutylammoniumchlorid, Tetramethylammoniumsulfat, Tetramethylammoniumchlorid und Tetrabutylammoniumbromid. Besonders bevorzugt ist Tetrabutylammoniumbromid.

Die Alkylierung lässt sich besonders vorteilhaft durchführen, wenn man als Alkylierungsmittel eine Verbindung der Formel VI verwendet, in der Y für Chlor, Brom, Jod, 4-Methylphenylsulfonyl, 4-Bromphenylsulfonyl oder Methylsulfonyl, insbesondere aber für Chlor, Brom oder Jod steht.

Nach dem erfindungsgemässen Verfahren werden besonders vorteilhaft Verbindungen der Formel I hergestellt, in denen $R_1$ für $-CH_2CR_2{=}CR_3R_4$ steht, wobei $R_2$, $R_3$ und $R_4$ vorzugsweise unabhängig voneinander für Wasserstoff, Chlor, Brom oder Methyl stehen.

Für die Hydrolyse geeignete starke Säuren sind beispielsweise Mineralsäuren wie Salzsäure, Schwefelsäure oder Phosphorsäure ; oder Sulfonsäuren wie Methansulfonsäure, Benzolsulfonsäure oder p-Toluolsulfonsäure. Besonders bevorzugt ist Salzsäure.

In einer besonders bevorzugten Variante des erfindungsgemässen Verfahrens setzt man Acetonitril und Ethanol in Toluol in Gegenwart von Chlorwasserstoff um, dosiert die erhaltene Suspension in eine eisgekühlte, wässrige Suspension von Natriumhydrogencarbonat ein, gibt der erhaltenen Mischung bei einer Temperatur von -20 bis -5°C eine wässrige Lösung von Hydroxylaminsulfat zu, trennt die wässrige Phase nach Erwärmung auf Raumtemperatur ab, gibt der erhaltenen Lösung des Acethydroximsäure-ethylesters in Toluol zunächst eine wässrige Lösung von Natriumhydroxid und dann trans-1,3-Dichlorpropen bei einer Temperatur von +50 bis +80°C zu, kühlt nach beendeter O-Alkylierung auf Raumtemperatur ab, trennt die wässrige Phase ab, hydrolysiert den gebildeten O-(trans-chlorallyl)-acethydroximsäure-ethylester durch Verrühren mit verdünnter Salzsäure, und trennt die das Hydrochlorid des O-(trans-chlorallyl)-hydroxylamins enthaltende wässrige Phase ab und isoliert dieses gegebenenfalls durch Verdampfen des Wassers.

Mit dem erfindungsgemässen Verfahren können die Verbindungen der Formel I mit geringem Aufwand aus leicht zugänglichen Ausgangsprodukten in guten Ausbeuten und hoher Reinheit hergestellt werden. Gegenüber dem aus USP 4,743,701 bekannten Verfahren fällt durch die Verwendung von Natriumhydrogencarbonat als Base nur die Hälfte der unerwünschten Ammoniumsalze als Nebenprodukt an.

Als besonders vorteilhaft hervorzuheben ist ferner, dass das erfindungsgemässe Verfahren ohne Lösungsmittelwechsel und ohne Isolierung von Zwischenprodukten durchgeführt wird. Dadurch wird der zeitliche und apparative Aufwand gegenüber den bekannten Verfahren erheblich reduziert.

Die folgenden Beispiele dienen der näheren Erläuterung des erfindungsgemässen Verfahrens.

Beispiel 1 : Herstellung von Acethydroximsäureethylester

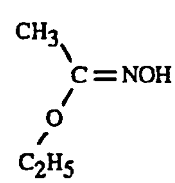

Zu einer Mischung aus 116,6 g Acetonitril und 125,6 g Ethanol abs. in 270 g Toluol leitet man innerhalb zwei Stunden bei einer Temperatur von +12 bis +15°C 102 g Chlorwasserstoff ein. Anschliessend wird die Reaktionsmischung 22 Stunden lang bei einer Temperatur von +20 bis +25°C gerührt. Nach Kühlen der Mischung auf -10°C fügt man 234,4 g Natriumhydrogencarbonat hinzu. Anschliessend lässt man innerhalb einer Stunde bei einer Temperatur von -10 bis -5°C eine Lösung von 207,6 g Hydroxylaminsulfat in 630 ml Wasser zutropfen, wobei $CO_2$-Entwicklung auftritt. Anschliessend lässt man das Reaktionsgemisch unter langsamer Erwärmung auf Raumtemperatur 90 Minuten lang rühren. Nach Abtrennen der wässrigen Phase wird die organische Phase mit 200 ml Wasser extrahiert. Nach Abtrennen der wässrigen Phase erhält man eine Lösung

aus 240,2 g Acethydroximsäureethylester (93,9 % d.Th.) in Toluol.

Beispiel 2 : Herstellung von O-(trans-chlorallyl)-hydroxylamin-Hydrochlorid (Verbindung Nr. 1.1)

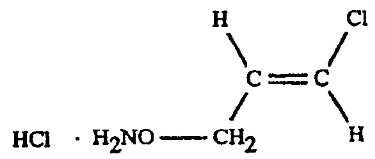

(1.1)

Zu einer Lösung von 173 g 30%ige Natronlauge in 173 ml Wasser gibt man innerhalb von 25 Minuten eine gemäss Beispiel 1 erhaltene Lösung von 277,6 g Acethydroximsäure-ethylester in Toluol (Gehalt : 39 %) tropfenweise hinzu. Anschliessend gibt man zu der entstandenen Suspension 3,2 g Tetrabutylammoniumbromid hinzu. Zu dieser Reaktionsmischung lässt man innerhalb von 30 Minuten bei einer Temperatur von +60 bis +65°C 115,7 g trans- 1,3-Dichlorpropen (Gehalt : 96 %) zutropfen. Nach Aufwärmen des Gemisches auf +70 bis +75°C für einen Zeitraum von 3 Stunden kühlt man die Reaktionsmischung auf Raumtemperatur und trennt die wässrige Phase anschliessend ab. Die organische Phase, die den O-(trans-chlorallyl)acethydroximsäure-ethylester (Verbindung Nr. 2.1) enthält, wird zweimal mit 250 ml Wasser extrahiert. Anschliessend gibt man die organische Phase innerhalb von 2 $\frac{1}{2}$ Stunden bei einer Temperatur von +20 bis +30°C zu einer Lösung von 102,4 g 32 %ige Salzsäure in 54 ml Wasser tropfenweise hinzu. Anschliessend erwärmt man das Reaktionsgemisch für einen Zeitraum von 2 $\frac{1}{2}$ Stunden auf eine Temperatur von +40 bis 45°C auf, lässt auf Raumtemperatur abkühlen und trennt die wässrige Phase ab. Man erhält 270,3 g (87,5 % d.Th. bezogen auf trans-1,3-Dichlorpropen) O-(trans-chlorallyl)-hydroxylamin (Verbindung Nr. 1.1) in Form seines Hydrochlorids (Gehalt : 46,6 %) in wässriger Lösung.

228 g der wässrigen Phase engt man bei 400 bis 500 Pa Druck und einer Temperatur von +60°C ein. Nach Trocknen des Rückstandes im Vakuum bei +50°C erhält man 108 g O-(trans-chlorallyl)-hydroxylamin (Verbindung Nr. 1.1) in Form seines Hydrochlorids mit einem Gehalt von 98,5 % und einem Schmelzpunkt von +164 bis +165°C. Nach Umkristallisation in Ethanol/Diethylether beträgt der Schmelzpunkt +182 bis +183°C.

Entsprechend den vorstehend beschriebenen Arbeitsweisen werden die in den Tabellen 1 und 2 aufgeführten Verbindungen hergestellt :

Tabelle 1:

$$H_2NOR_1 \cdot HCl \qquad (I)$$

| Verbindung Nr. | $R_1$ | Smp [°C] |
|---|---|---|
| 1.1 | 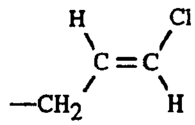 | +182 bis +183 |
| 1.2 | 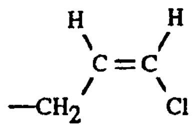 | +175 bis +176 |
| 1.3 | 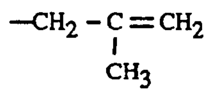 | +165 |
| 1.4 | 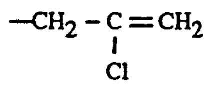 | |
| 1.5 | 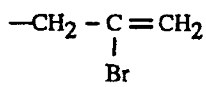 | |
| 1.6 | 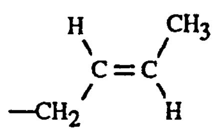 | +158 bis +160 |
| 1.7 | $-CH_2-CBr=CBrH$ | (cis/trans-Gemisch) |
| 1.8 | $-CH_2-CH=CCH_3Cl$ | (cis/trans-Gemisch) |
| 1.9 | $-CH_2-C\equiv CH$ | +152 bis +153 |
| 1.10 | $-CH_3$ | +147 bis +148 |
| 1.11 | $-CH_2CH_2Cl$ | +180 bis +181 |

Tabelle 2: 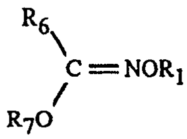 (VII)

| Verbindung Nr. | $R_7$ | $R_6$ | $R_1$ | Sdp. [°C]/ Druck [Pa] |
|---|---|---|---|---|
| 2.1 | $C_2H_5$ | $CH_3$ | 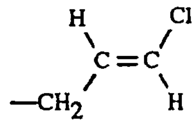 | 49-50°/200 |
| 2.2 | $C_2H_5$ | $CH_3$ | 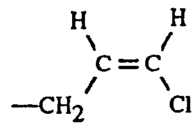 | 68-69°/800 |
| 2.3 | $C_2H_5$ | $CH_3$ | 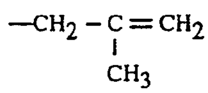 | 78°/5000 |
| 2.4 | $C_2H_5$ | $CH_3$ | 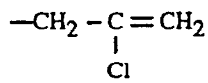 | 79°/2200 |
| 2.5 | $C_2H_5$ | $CH_3$ | 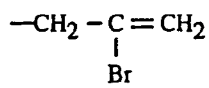 | 77°/1100 |
| 2.6 | $C_2H_5$ | $CH_3$ | 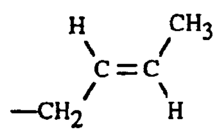 | 60-61°/1000 |
| 2.7 | $C_2H_5$ | $CH_3$ | $-CH_2CBr{=}CBrH$ | 89-90°/40 (cis/trans-Gemisch) |
| 2.8 | $C_2H_5$ | $CH_3$ | $-CH_2-CH{=}CCH_3Cl$ | 93°/1500 (cis/trans-Gemisch) |
| 2.9 | $C_2H_5$ | $CH_3$ | $-CH_2-C{\equiv}CH$ | 40°/500 |

Tabelle 2: Fortsetzung

| Verbindung Nr. | $R_7$ | $R_6$ | $R_1$ | Sdp. [°C]/ Druck [Pa] |
|---|---|---|---|---|
| 2.10 | $C_2H_5$ | $C_2H_5$ | 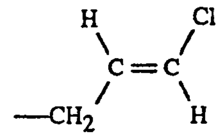 | |
| 2.11 | $C_2H_5$ | i-$C_3H_8$ | 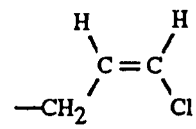 | |
| 2.12 | $C_2H_5$ | Phenyl | 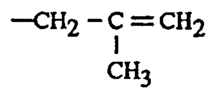 | |
| 2.13 | $C_2H_5$ | Benzyl | 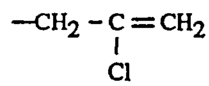 | |
| 2.14 | $CH_3$ | $CH_3$ | 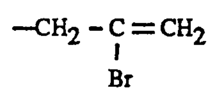 | |
| 2.15 | i-$C_3H_8$ | $CH_3$ | 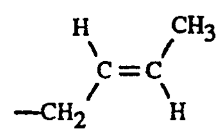 | |
| 2.16 | Benzyl | $CH_3$ | $-CH_2CBr{=}CBrH$ | |
| 2.17 | $C_2H_5$ | $CH_3$ | $-CH_3$ | +106 bis +107 |
| 2.18 | $C_2H_5$ | $CH_3$ | $-CH_2CH_2Cl$ | + 41/250 |
| 2.19 | $CH_3OCH_2CH_2$- | $CH_3$ | $-CH_2-C\equiv CH$ | |

**Patentansprüche**

1. Verfahren zur Herstellung von O-substituierten Hydroxylaminen der Formel I

$$H_2NOR_1 \quad (I)$$

worin

$R_1$ $C_1$-$C_6$-Alkyl, $C_1$-$C_6$-Halogenalkyl, $-CH_2CR_2{=}CR_3R_4$ oder $-CH_2C{\equiv}CR_5$ ;

$R_2$ Wasserstoff, Halogen oder Methyl ;

$R_3$ Wasserstoff, Halogen oder Methyl ;

$R_4$ Wasserstoff, Halogen oder Methyl ; und
$R_5$ Wasserstoff, Methyl oder Ethyl bedeuten,
Umsetzung eines Nitrils mit einem Alkohol in Gegenwart von Halogenwasserstoff zum Iminoester-Hydrohalogenid, dessen weitere Umsetzung mit einem Hydroxylaminsalz in Gegenwart einer Base zum Hydroximsäureester, dessen weitere Umsetzung mit einem Alkylierungsmittel zum Oximether und dessen hydrolytische Spaltung, dadurch gekennzeichnet, dass man ein Nitril der Formel II

$$R_6\text{-CN} \quad (II)$$

worin $R_6$ für $C_1$-$C_4$-Alkyl, Phenyl oder Benzyl steht, mit einem Alkohol der Formel III

$$R_7\text{-OH} \quad (III)$$

worin $R_7$ für $C_1$-$C_5$-Alkyl, Benzyl oder $C_1$-$C_4$-Alkoxyethyl steht, in einem organischen mit Wasser nicht oder nur sehr begrenzt mischbaren Lösungsmittel in Gegenwart von Halogenwasserstoff HX umsetzt, die erhaltene Suspension des Iminoester-Hydrohalogenids der Formel IV

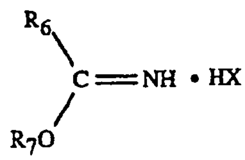

(IV)

worin $R_6$ und $R_7$ die oben angegebene Bedeutung haben und X für Chlor, Brom oder Jod steht, bei einer Temperatur von -3 bis +5°C in eine wässrige Suspension eines Carbonats oder Hydrogencarbonats eindosiert, der erhaltenen Mischung bei einer Temperatur von -20 bis -5°C eine wässrige Lösung eines Hydroxylaminsalzes zugibt, die wässrige Phase nach Erwärmung auf Raumtemperatur abtrennt, der erhaltenen Lösung des Hydroximsäureesters der Formel V

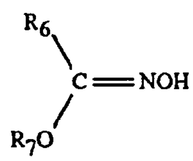

(V)

worin $R_6$ und $R_7$ die oben angegebene Bedeutung haben, in einem organischen Lösungsmittel zunächst eine wässrige Lösung einer starken Base und dann ein Alkylierungsmittel der Formel VI

$$Y\text{-}R_1 \quad (VI)$$

worin $R_1$ die oben angegebene Bedeutung hat und Y für Chlor, Brom, Jod oder $-O-SO_2R_8$, und $R_8$ für $C_1$-$C_4$-Alkyl, $C_1$-$C_4$-Halogenalkyl, Phenyl oder durch Phenyl substituiertes $C_1$-$C_4$-Alkyl steht, wobei der Phenylring seinerseits noch ein- bis dreifach durch Halogen oder $C_1$-$C_4$-Alkyl substituiert sein kann und Y auch für $-OSO_2OR_1$ steht, wenn $R_1$ $C_1$-$C_2$-Alkyl bedeutet, bei Raumtemperatur bis zur Siedetemperatur des Reaktionsgemisches zugibt, nach beendeter O-Alkylierung auf Raumtemperatur abkühlt, die wässrige Phase abtrennt, den gebildeten Oximether der Formel VII

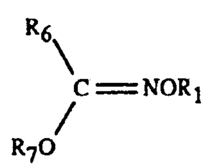

(VII)

worin $R_1$, $R_6$ und $R_7$ die oben angegebene Bedeutung haben, durch Verrühren mit einer wässrigen Lösung einer starken Säure hydrolysiert und die das Salz des O-substituierten Hydroxylamins der Formel I enthaltende wässrige Phase abtrennt, und dieses gegebenenfalls durch Verdampfen des Wassers isoliert.

2. Verfahren nach Anspruch 1, dadurch gekennzeichnet, dass man als organisches mit Wasser nicht oder nur sehr begrenzt mischbares Lösungsmittel Toluol, Diethylether, Xylol, Cyclohexan, Nitrobenzol, Dichlorethan, Hexan, Dichlormethan oder Trichlormethan verwendet.

3. Verfahren nach Anspruch 2, dadurch gekennzeichnet, dass man Toluol verwendet.

4. Verfahren nach Anspruch 1, dadurch gekennzeichnet, dass man als Hydroxylaminsalz das Hydroxylamin-Hydrochlorid oder das Hydroxylaminsulfat verwendet.

5. Verfahren nach Anspruch 1, dadurch gekennzeichnet, dass Y für Chlor, Brom oder Jod oder für 4-Methylphenylsulfonyl, 4-Bromphenylsulfonyl oder Methylsulfonyl steht.

6. Verfahren nach Anspruch 1, dadurch gekennzeichnet, dass X für Chlor steht.

7. Verfahren nach Anspruch 1, dadurch gekennzeichnet, dass man als starke Base Natriumhydroxid oder Natriumcarbonat verwendet.

8. Verfahren nach Anspruch 1, dadurch gekennzeichnet, dass man die Alkylierung in Gegenwart einer katalytischen Menge eines Phasentransferkatalysators durchführt.

9. Verfahren nach Anspruch 8, dadurch gekennzeichnet, dass man als Phasentransferkatalysator Tetrabutylammoniumbromid verwendet.

10. Verfahren nach Anspruch 1, dadurch gekennzeichnet, dass man als Alkylierungsmittel eine Verbindung der Formel VI einsetzt, in der $R_1$ für $-CH_2-CR_2=CR_3R_4$ steht, wobei $R_2$, $R_3$ und $R_4$ die in Anspruch 1 angegebene Bedeutung haben.

11. Verfahren nach Anspruch 10, dadurch gekennzeichnet, dass $R_2$, $R_3$ und $R_4$ unabhängig voneinander für Wasserstoff, Chlor, Brom oder Methyl stehen.

12. Verfahren nach Anspruch 1, dadurch gekennzeichnet, dass man in die Suspension des Iminoester-Hydrohalogenids der Formel IV eine wässrige Suspension von Natriumhydrogencarbonat, Kaliumhydrogencarbonat, Natriumcarbonat oder Kaliumcarbonat eindosiert.

13. Verfahren nach Anspruch 12, dadurch gekennzeichnet, dass man eine wässrige Suspension von Natriumhydrogencarbonat eindosiert.

14. Verfahren nach Anspruch 1, dadurch gekennzeichnet, dass man als Nitril der Formel II Acetonitril oder Ethylnitril und als Alkohol der Formel III Methanol oder Ethanol einsetzt.

15. Verfahren nach Anspruch 1, dadurch gekennzeichnet, dass man Acetonitril und Ethanol in Toluol in Gegenwart von Chlorwasserstoff umsetzt, die erhaltene Suspension in eine eisgekühlte, wässrige Suspension von Natriumhydrogencarbonat eindosiert, der erhaltenen Mischung bei einer Temperatur von -20 bis -5°C eine wässrige Lösung von Hydroxylaminsulfat zugibt, die wässrige Phase nach Erwärmung auf Raumtemperatur abtrennt, der erhaltenen Lösung des Acethydroximsäure-ethylesters in Toluol zunächst eine wässrige Lösung von Natriumhydroxid und dann trans-1,3-Dichlorpropen bei einer Temperatur von +50 bis +80°C zugibt, nach beendeter O-Alkylierung auf Raumtemperatur abkühlt, die wässrige Phase abtrennt, den gebildeten O-(trans-chlorallyl)-acethydroximsäure-ethylester durch Verrühren mit verdünnter Salzsäure hydrolysiert, und die das Hydrochlorid des O-(trans-chlorallyl)-hydroxylamins enthaltende wässrige Phase abtrennt und dieses gegebenenfalls durch Verdampfen des Wassers isoliert.

16. Verbindungen der Formel VII

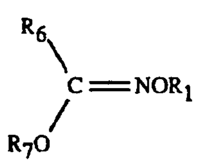

(VII)

worin
$R_7$ $C_1$-$C_5$-Alkyl, Benzyl oder $C_1$-$C_4$-Alkoxyethyl ;
$R_6$ $C_1$-$C_4$-Alkyl, Phenyl oder Benzyl ;
$R_1$ $-CH_2CR_2=CR_3R_4$ oder $-CH_2C\equiv CR_5$ ;

$R_2$ Wasserstoff, Halogen oder Methyl ;
$R_3$ Wasserstoff, Halogen oder Methyl ;
$R_4$ Wasserstoff, Halogen oder Methyl und
$R_5$ Wasserstoff, Methyl oder Ethyl bedeuten, mit der Massgabe, dass $R_2$, $R_3$ und $R_4$ nicht gleichzeitig für Wasserstoff stehen.

**17.** Verbindungen der Formel VII nach Anspruch 16, dadurch gekennzeichnet, dass $R_7$ und $R_6$ unabhängig voneinander für $C_1$-$C_2$-Alkyl stehen.

**18.** Verbindungen der Formel VII nach Anspruch 16, dadurch gekennzeichnet, dass $R_1$ für $-CH_2CR_2{=}CR_3R_4$ steht.

**19.** Verbindungen der Formel VII nach Anspruch 16, dadurch gekennzeichnet, dass $R_2$, $R_3$ und $R_4$ unabhängig voneinander für Wasserstoff, Chlor, Brom oder Methyl stehen.

**20.** Verbindungen der Formel VII nach Anspruch 15, dadurch gekennzeichnet, dass $R_1$ für Chlorallyl steht.

**21.** O-(trans-chlorallyl)-acethydroximinsäure-ethylester nach Anspruch 16.

**Patentansprüche Für folgenden Vertragsstaat : ES**

**1.** Verfahren zur Herstellung von O-substituierten Hydroxylaminen der Formel I

$$H_2NOR_1 \quad (I)$$

worin
$R_1$ $C_1$-$C_6$-Alkyl, $C_1$-$C_6$-Halogenalkyl, $-CH_2CR_2{=}CR_3R_4$ oder $-CH_2C{\equiv}CR_5$ ;
$R_2$ Wasserstoff, Halogen oder Methyl ;
$R_3$ Wasserstoff, Halogen oder Methyl ;
$R_4$ Wasserstoff, Halogen oder Methyl ; und
$R_5$ Wasserstoff, Methyl oder Ethyl bedeuten,
Umsetzung eines Nitrils mit einem Alkohol in Gegenwart von Halogenwasserstoff zum Iminoester-Hydrohalogenid, dessen weitere Umsetzung mit einem Hydroxylaminsalz in Gegenwart einer Base zum Hydroximsäureester, dessen weitere Umsetzung mit einem Alkylierungsmittel zum Oximether und des sen hydrolytische Spaltung, dadurch gekennzeichnet, dass man ein Nitril der Formel II

$$R_6\text{-}CN \quad (II)$$

worin $R_6$ für $C_1$-$C_4$-Alkyl, Phenyl oder Benzyl steht, mit einem Alkohol der Formel III

$$R_7\text{-}OH \quad (III)$$

worin $R_7$ für $C_1$-$C_5$-Alkyl, Benzyl oder $C_1$-$C_4$-Alkoxyethyl steht, in einem organischen mit Wasser nicht oder nur sehr begrenzt mischbaren Lösungsmittel in Gegenwart von Halogenwasserstoff HX umsetzt, die erhaltene Suspension des Iminoester-Hydrohalogenids der Formel IV

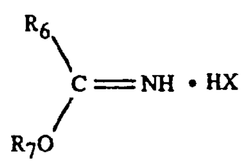

(IV)

worin $R_6$ und $R_7$ die oben angegebene Bedeutung haben und X für Chlor, Brom oder Jod steht, bei einer Temperatur von -3 bis +5°C in eine wässrige Suspension eines Carbonats oder Hydrogencarbonats eindosiert, der erhaltenen Mischung bei einer Temperatur von -20 bis -5°C eine wässrige Lösung eines Hydroxylaminsalzes zugibt, die wässrige Phase nach Erwärmung auf Raumtemperatur abtrennt, der erhaltenen Lösung des Hydroximsäureesters der Formel V

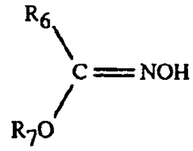

(V)

worin $R_6$ und $R_7$ die oben angegebene Bedeutung haben, in einem organischen Lösungsmittel zunächst eine wässrige Lösung einer starken Base und dann ein Alkylierungsmittel der Formel VI

$$Y\text{-}R_1 \quad (VI)$$

worin $R_1$ die oben angegebene Bedeutung hat und Y für Chlor, Brom, Jod oder $-O-SO_2R_8$, und $R_8$ für $C_1$-$C_4$-Alkyl, $C_1$-$C_4$-Halogenalkyl, Phenyl oder durch Phenyl substituiertes $C_1$-$C_4$-Alkyl steht, wobei der Phenylring seinerseits noch ein- bis dreifach durch Halogen oder $C_1$-$C_4$-Alkyl substituiert sein kann und Y auch für $-OSO_2OR_1$ steht, wenn $R_1$ $C_1$-$C_2$-Alkyl bedeutet, bei Raumtemperatur bis zur Siedetemperatur des Reaktionsgemisches zugibt, nach beendeter O-Alkylierung auf Raumtemperatur abkühlt, die wässrige Phase abtrennt, den gebildeten Oximether der Formel VII

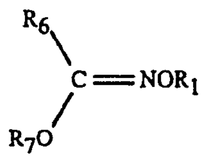

(VII)

worin $R_1$, $R_6$ und $R_7$ die oben angegebene Bedeutung haben, durch Verrühren mit einer wässrigen Lösung einer starken Säure hydrolysiert und die das Salz des O-substituierten Hydroxylamins der Formel I enthaltende wässrige Phase abtrennt, und dieses gegebenenfalls durch Verdampfen des Wassers isoliert.

2. Verfahren nach Anspruch 1, dadurch gekennzeichnet, dass man als organisches mit Wasser nicht oder nur sehr begrenzt mischbares Lösungsmittel Toluol, Diethylether, Xylol, Cyclohexan, Nitrobenzol, Dichlorethan, Hexan, Dichlormethan oder Trichlormethan verwendet.

3. Verfahren nach Anspruch 2, dadurch gekennzeichnet, dass man Toluol verwendet.

4. Verfahren nach Anspruch 1, dadurch gekennzeichnet, dass man als Hydroxylaminsalz das Hydroxylamin-Hydrochlorid oder das Hydroxylaminsulfat verwendet.

5. Verfahren nach Anspruch 1, dadurch gekennzeichnet, dass Y für Chlor, Brom oder Jod oder für 4-Methylphenylsulfonyl, 4-Bromphenylsulfonyl oder Methylsulfonyl steht.

6. Verfahren nach Anspruch 1, dadurch gekennzeichnet, dass X für Chlor steht.

7. Verfahren nach Anspruch 1, dadurch gekennzeichnet, dass man als starke Base Natriumhydroxid oder Natriumcarbonat verwendet.

8. Verfahren nach Anspruch 1, dadurch gekennzeichnet, dass man die Alkylierung in Gegenwart einer katalytischen Menge eines Phasentransferkatalysators durchführt.

9. Verfahren nach Anspruch 8, dadurch gekennzeichnet, dass man als Phasentransferkatalysator Tetrabutylammoniumbromid verwendet.

10. Verfahren nach Anspruch 1, dadurch gekennzeichnet, dass man als Alkylierungsmittel eine Verbindung der Formel VI einsetzt, in der $R_1$ für $-CH_2-CR_2=CR_3R_4$ steht, wobei $R_2$, $R_3$ und $R_4$ die in Anspruch 1 angegebene Bedeutung haben.

11. Verfahren nach Anspruch 10, dadurch gekennzeichnet, dass $R_2$, $R_3$ und $R_4$ unabhängig voneinander für Wasserstoff, Chlor, Brom oder Methyl stehen.

**12.** Verfahren nach Anspruch 1, dadurch gekennzeichnet, dass man in die Suspension des Iminoester-Hydrohalogenids der Formel IV eine wässrige Suspension von Natriumhydrogencarbonat, Kaliumhydrogencarbonat, Natriumcarbonat oder Kaliumcarbonat eindosiert.

**13.** Verfahren nach Anspruch 12, dadurch gekennzeichnet, dass man eine wässrige Suspension von Natriumhydrogencarbonat eindosiert.

**14.** Verfahren nach Anspruch 1, dadurch gekennzeichnet, dass man als Nitril der Formel II Acetonitril oder Ethylnitril und als Alkohol der Formel III Methanol oder Ethanol einsetzt.

**15.** Verfahren nach Anspruch 1, dadurch gekennzeichnet, dass man Acetonitril und Ethanol in Toluol in Gegenwart von Chlorwasserstoff umsetzt, die erhaltene Suspension in eine eisgekühlte, wässrige Suspension von Natriumhydrogencarbonat eindosiert, der erhaltenen Mischung bei einer Temperatur von -20 bis -5°C eine wässrige Lösung von Hydroxylaminsulfat zugibt, die wässrige Phase nach Erwärmung auf Raumtemperatur abtrennt, der erhaltenen Lösung des Acethydroximsäure-ethylesters in Toluol zunächst eine wässrige Lösung von Natriumhydroxid und dann trans-1,3-Dichlorpropen bei einer Temperatur von +50 bis +80°C zugibt, nach beendeter O-Alkylierung auf Raumtemperatur abkühlt, die wässrige Phase abtrennt, den gebildeten O-(trans-chlorallyl)-acethydroximsäure-ethylester durch Verrühren mit verdünnter Salzsäure hydrolysiert, und die das Hydrochlorid des O-(trans-chlorallyl)-hydroxylamins enthaltende wässrige Phase abtrennt und dieses gegebenenfalls durch Verdampfen des Wassers isoliert.

Europäisches Patentamt

# EUROPÄISCHER RECHERCHENBERICHT

Nummer der Anmeldung

EP 91810055.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| Y | DE - A1 - 3 100 728 (BAYER AG) * Beispiele 4,10 * -- | 1,2,5, 6,12 | C 07 C 239/20<br>C 07 C 259/04 |
| Y | PATENT ABSTRACTS OF JAPAN, unexamined applications, C field, Band 10, Nr. 13, 18. Jänner 1986 THE PATENT OFFICE JAPANESE GOVERNMENT Seite 149 C 323 * Kokai-Nr. 60-169 446 (MITSUI SEKIYU KAGAKU KOGYO) * -- | 1,2,5, 6,12 | |
| D,A | DE - A1 - 2 651 083 (HOECHST) * Beispiel 1; Anspruch 3 * -- | 1,16 | |
| A | WO - A1 - 88/04 291 (ALLIED CORPORATION) * Ansprüche 1-19; Beispiel 1 * | 1,4,6, 14 | RECHERCHIERTE SACHGEBIETE (Int Cl⁵) |
| D,A | & US-A-4 743 701 ---- | | C 07 C 239/00<br>C 07 C 259/00 |

Der vorliegende Recherchenbericht wurde für alle Patentansprüche erstellt.

| Recherchenort | Abschlußdatum der Recherche | Prüfer |
|---|---|---|
| VIENNA | 07-05-1991 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82